Europäisches Patentamt

European Patent Office

Office européen des brevets

(11) Numéro de publication: **0 269 503 B1**

(12) **FASCICULE DE BREVET EUROPEEN**

(45) Date de publication de fascicule du brevet: **26.08.92**

(51) Int. Cl.5: **C01B 35/12**, C01B 33/34, C01B 33/20, B01J 29/04, B01J 29/28, C07C 2/12, C07C 2/08

(21) Numéro de dépôt: **87402507.5**

(22) Date de dépôt: **06.11.87**

(54) **Nouvelles ferriérites, leur procédé de préparation et leur utilisation.**

(30) Priorité: **21.11.86 FR 8616362**

(43) Date de publication de la demande:
**01.06.88 Bulletin 88/22**

(45) Mention de la délivrance du brevet:
**26.08.92 Bulletin 92/35**

(84) Etats contractants désignés:
**AT BE DE ES FR GB IT NL**

(56) Documents cités:
**EP-A- 0 049 386
US-A- 4 016 245
US-A- 4 046 859
US-A- 4 107 195**

(73) Titulaire: **INSTITUT FRANCAIS DU PETROLE
4, Avenue de Bois Préau
F-92502 Rueil-Malmaison(FR)**

(72) Inventeur: **Guth, Jean-Louis
59, rue Bellevue
Brunstatt F-68200 Mulhouse(FR)**
Inventeur: **Faust, Anne-Catherine
23, rue de Bourgogne
F-68100 Mulhouse(FR)**
Inventeur: **Raatz, Francis
17, rue Michelet
F-92500 Rueil Malmaison(FR)**
Inventeur: **Lamblin, Jean-Marc
96, rue de Belfort
F-68200 Mulhouse-Dornach(FR)**

**EP 0 269 503 B1**

## Description

La présente invention concerne de nouvelles compositions de ferriérites, leur méthode de préparation, leur utilisation en séparation par adsorption et comme catalyseur ou support de catalyseur de conversion d'hydrocarbures.

La ferriérite est une zéolithe naturelle de formule approchée $(Na, Mg)_{x/3} (AlO_2)_x(SiO_2)_{36-x} \cdot 8H_2O$ caractérisée par une valeur de x proche de 6. La structure d'une zéolithe cristallisée est formée d'une charpente résultant de l'enchaînement de tétraèdres $TO_{4/2}$, chaque oxygène étant commun à deux tétraèdres. Le plus souvent T est du silicium ou de l'aluminium. Mais d'autres éléments trivalents comme le bore, le gallium ou le fer peuvent être introduits à la place de l'aluminium dans les tétraèdres $TO_4$ de la charpente de certaines zéolithes. La présence de ces éléments trivalents à côté du silicium tétravalent confère des charges négatives à la charpente, charges qui sont neutralisées par des cations de compensations. Le volume microporeux laissé libre par la charpente est occupé par les cations de compensations échangeables et différentes espèces chimiques comme de l'eau, des amines, des alcools, des sels, des bases etc... Il faut noter le rôle important joué par toutes ces espèces qui par leur insertion au cours de la formation des zéolithes, sont à l'origine de leur structure microporeuse. En effet leurs formes, leurs dimensions et les interactions qu'elles produisent permettent l'édification de charpentes peu denses comportant des interstices variables en volume et en forme.

L'utilisation des zéolithes comme solides microporeux en adsorption sélective ou en catalyse nécessite l'élimination au moins partielle par chauffage ou calcination des espèces insérées pendant la synthèse. Chaque type de zéolithe possède alors une structure microporeuse distincte liée à la géométrie de la charpente ainsi qu'au nombre et à la taille des cations de compensation présents. La variation des dimensions et formes des canaux et cavités d'un type à l'autre entraîne des changements des propriétés adsorbantes. Seules les molécules ayant certaines dimensions et formes sont capables d'entrer dans les pores d'une zéolithe donnée. En raison de ces caractéristiques remarquables les zéolithes conviennent tout particulièrement pour la purification ou la séparation de mélanges gazeux ou liquides comme par exemple la séparation d'hydrocarbures par adsorption sélective.

La composition chimique avec la nature des éléments présents dans les tétraèdres $TO_{4/2}$ et la nature des cations de compensation est également un facteur important intervenant dans la sélectivité de l'adsorption et surtout dans l'activité catalytique de ces produits. Ceci s'explique par la nature et l'intensité des interactions entre les zéolithes et les molécules adsorbées sur leur surface interne. Elles sont utilisées comme catalyseurs ou support de catalyseurs dans le craquage et la modification d'hydrocarbures ainsi que dans l'élaboration de nombreuses molécules.

Parmi les différents types de zéolithes utilisables industriellement dans des procédés de séparation ou catalytiques, la ferriérite occupe déjà une place importante. Avec un rapport Si/Al relativement élevé, elle offre une structure ayant une bonne stabilité thermique. Pour les mêmes raisons elle possède une acidité forte lorsque les cations de compensation sont des protons $H^+$ et sa stabilité reste convenable. Enfin la géométrie de son système microporeux et la dimension des micropores (4,3 × 5,5 Å dans la direction (001) et 3,4 × 4,8 Å dans la direction (010) avec une interconnexion des deux systèmes de canaux) en fait un solide microporeux ayant une bonne sélectivité en adsorption et catalyse.

On connait des gisements de ferriérites naturelles. Mais les applications industrielles de ces produits sont limitées par la qualité variable des produits et la présence d'impuretés constituant des charges indésirables dans certains procédés. D'autre part certaines applications nécessitent des rapports Si/Al plus élevés que celui, situé entre 4 et 7, des ferriérites naturelles. Il est bien entendu possible en principe d'augmenter ce rapport par différents traitements chimiques mais ces procédés sont longs et coûteux. Par ailleurs il a été montré que la désalumination de la ferriérite par les traitements post synthèse connus dans l'art antérieur s'avère très difficile, voire impossible (G. FERRE, Thèse Docteur-Ingénieur ENSPM, 1986).

Ces problèmes sont à l'origine d'importantes recherches en vue de synthétiser des ferriérites ayant les qualités et propriétés souhaitées. Il existe actuellement de nombreux brevets et publications décrivant ces synthèses. On peut citer une publication de R.M. BARRER et D.J. MARSHALL (J. Chem. Soc. 1964 p. 485) et les brevets suivants : FR 2228721 (1974) ; US 3966883 (1976) ; Ger offen 2548697 (1976) ; US 3933974 (1976) ; US 4016245 (1977) ; US 4017590 (1977) ; US 4088739 (1978) ; EP 12473 (1980) ; US 4259306 (1982) ; EP 55529 (1982) ; EP 49386 (1982) ; JP 5973423 (1984) ; JP 60191017 (1985) ; JP 60141617 (1985).

Les ferriérites synthétiques décrites dans la littérature sont toutes préparées par traitement hydrothermal d'un mélange aqueux basique contenant une source de silice et d'alumine. Pour obtenir le pH basique (>10) nécessaire à la dissolution des sources de silice et d'alumine et à la cristallisation de la zéolithe on utilise généralement une ou plusieurs bases minérales comme NaOH et (ou) des silicates et aluminates

2

alcalins. A ces bases on associe dans de nombreux cas des amines ou ammonium quaternaires, ceci dans le but de diriger la cristallisation du gel vers la ferriérite et de favoriser l'augmentation du rapport Si/Al dans la zéolithe, comme l'illustrent les brevets US 4 046 859 et US 4 107 195.

Cette manière de procéder présente plusieurs inconvénients. Les zéolithes ayant des charpentes peu denses comme la ferriérite sont généralement métastables dans leur milieu de formation basique et de ce fait difficiles à obtenir à l'état très pur. Elles sont souvent accompagnées par des phases plus denses et plus stables. Cette difficulté s'accroît lorsque les quantités à préparer augmentent c'est-à-dire lorsqu'on passe de l'échelle laboratoire à l'échelle industrielle.

D'autre part ces milieux basiques ne sont pas favorables à la cristallisation de phases très riches en silice. Il est en effet bien connu que l'augmentation du pH dans le milieu de synthèse produit une diminution du rapport Si/Al dans la zéolithe qui cristallise. On peut lier à ceci la solubilité d'autant plus importante en milieu basique que le rapport Si/Al est élevé avec comme conséquence une chute du rendement des préparations. La métastabilité des zéolithes nécessite des milieux très fortement sursaturés pour obtenir leur cristallisation ce qui produit une nucléation rapide au détriment de la croissance et conduit à des cristaux généralement de petite taille.

De nombreuses applications de la ferriérite, en particulier dans la catalyse acide, nécessitent une zéolithe sous une forme protonée et complètement débarassée de ses cations alcalins introduits en tant que cations de compensation lors de la synthèse. On peut y accéder par des procédés d'échanges d'ions répétés et longs avec des cations $NH_4^+$ suivis de calcination pour les décomposer en cations $H^+$. Cette étape d'échange d'ions pourrait être éliminée si on avait la possibilité de remplacer les cations alcalins par des cations $NH_4^+$ lors de la synthèse. Or ceci n'est pas possible lorsque le pH est supérieur à environ 10, $NH_4^+$ étant dans ces conditions transformé en $NH_3$. D'autre part, les synthèses effectuées à des pH où le cation $NH_4^+$ est stable, sont difficiles et longues à cause de la faible solubilité des sources de silice et d'alumine à ces bas pH.

L'invention a pour objet une ferriérite synthétique de formule chimique approchée suivante :

$$M_{x/n} (T^{III}O_2)_x (Si^{IV}O_2)_{36-x}$$

où $T^{III}$ représente au moins un élément choisi dans le groupe du bore ($B^{III}$), de l'aluminium ($Al^{III}$), du gallium ($Ga^{III}$) et du fer ($Fe^{III}$),

x est compris entre environ 0,05 et 6,6.

M est un proton résultant de la décomposition thermique de cations azotés, et/ou au moins un cation non décomposable issu du milieu réactionnel, par exemple un cation de métaux alcalins, alcalino-terreux, de métaux de transition ou autres métaux précisés ci-après, et/ou un cation introduit par des procédés d'échange d'ions.

n est la valence de M.

La ferriérite de la présente invention est également caractérisée par :

- un diagramme de diffraction X représenté dans le tableau 1,
- une teneur en fluor comprise entre environ 0,01 % et 1,6 % poids,
- un rapport molaire $Si^{IV}/T^{III}$ au moins égal à environ 4,75 et au plus égal à environ 700.

Les nouvelles compositions de ferriérites selon l'invention ont un rapport molaire $Si^{IV}/T^{III}$ compris de préférence entre 7 et 500, de manière encore plus préférée compris entre 20 et 300 et une teneur en fluor de préférence comprise entre 0,20 % et 1,0 % poids.

La présence de fluor dans la ferriérite de la présente invention modifie ses propriétés catalytiques notamment et permet d'obtenir des solides dont les performances sont différentes de celles connues dans l'art antérieur.

Dans le cas où des cations métalliques ou alcalino-terreux sont introduits lors de la synthèse, ces ions peuvent être aisément éliminés par des opérations d'échange ionique classique et remplacés soit directement ($H^+$) soit indirectement ($NH_4^+$) par des protons.

Dans les applications, on cherchera à conserver l'élément fluor au sein du solide mais on peut également si nécessaire éliminer en partie le fluor. L'élimination du fluor peut être réalisée en traitant pendant plusieurs heures à l'autoclave entre 100 et 200°C la ferriérite dans une solution diluée d'ammoniaque (rapport volume de solution sur poids de solide compris entre 5 et 20 $cm^3g^{-1}$).

L'invention a également pour objet un nouveau procédé de préparation de zéolithe de type ferriérite où les inconvénients qui ont été décrits plus haut (notamment rapports Si/Al faibles, présence obligatoire de cations non décomposables) n'existent plus. Selon ce nouveau procédé les synthèses sont effectuées dans des milieux aqueux ayant un pH inférieur à environ 10 et contenant des anions fluorure. Ces derniers remplacent les anions $OH^-$ des milieux basiques classiques et permettent la mobilisation dans la phase aqueuse des différents éléments dont le silicium entrant dans les tétraèdres $TO_{4/2}$ de la charpente. Le nouveau procédé de synthèse est caractérisé en ce que :

- On prépare un mélange réactionnel en solution à un pH inférieur à environ 10, comprenant notamment de l'eau, une source d'oxyde de silicium, une source d'au moins un oxyde de métal trivalent T choisi dans le groupe constitué par le bore, le gallium, l'aluminium et le fer, une source d'ions fluorures $F^-$ et une source d'au moins un agent structurant fournissant des cations organiques contenant de l'azote ; l'agent structurant est choisi parmi les monoamines, diamines, triamines, aliphatiques linéaires primaires ou secondaires, et les cations ammonium dérivant par protonation desdites amines, ledit agent ayant un nombre total d'atomes de carbone et d'azote (C-N) de 3 à 8 et de préférence de 3 à 5. Ledit mélange a une composition en termes de rapports molaires comprise dans les intervalles suivants :

  $Si^{IV}/\Sigma T^{III}$ : 2 - 400 ($T^{III}$ = B, Al, Ga, Fe)

  $F^-/Si^{IV}$ 0,1 - 3

  Structurant organique/$Si^{IV}$ : 0,1 - 4

  $H_2O/Si^{IV}$ : 4 - 200.

- On chauffe ledit mélange à une température au plus égale à environ 270°C, de manière avanageuse entre 120 et 260°C et de préférence entre 150°C et 240°C, pendant une durée suffisante pour obtenir des cristaux de ferriérite et,

- On calcine lesdits cristaux à une température supérieure à 400°C et de préférence comprise entre 500 et 600°C. L'étape de calcination a pour but l'élimination des cations organiques ou ammonium contenus dans le solide brut de synthèse.

On obtient ainsi des ferriérites sous forme $H^+$ par simple calcination de produits résultants de la synthèse. Ces ferriérites acides peuvent être mises en oeuvre telles quelles dans de nombreux procédés catalytiques employant des catalyseurs acides solides ou elles peuvent être échangées très facilement par tous les éléments donnant en solution aqueuse des cations stables. On peut citer à titre d'exemple $Pt^{2+}$, $Fe^{2+}$, $Fe^{3+}$, les cations de terres rares etc...

En ce qui concerne l'étape de synthèse proprement dite, on peut opérer de manière préférée à un pH compris entre 3,5 et 10. D'excellents résultats ont été obtenus notamment des ferriérites ayant des rapports silice/oxyde de métal T très élevés aussi bien avec l'aluminium, le bore, le gallium ou le fer, seuls ou en associations entre eux, lorsque le pH était compris entre environ 4,5 et 9.

Dans le mélange réactionnel de départ en solution aqueuse, on peut choisir de préférence une composition dont les rapports molaires sont compris dans les intervalles suivants :

$Si^{IV}/\Sigma T^{III}$ 4 - 150 ($T^{III}$ = B, Al, Ga, Fe)

$F^-/Si^{IV}$ 0,25 - 2,5

Structurant organique/$Si^{IV}$ 0.25 - 2.5

$H_2O/Si^{IV}$ 6 - 80.

Après l'étape de calcination on peut introduire dans les ferriérites par des méthodes d'échange d'ions bien connues, au moins un élément du tableau périodique dont les cations peuvent être préparés en milieu aqueux et choisis dans le groupe formé par les groupes $II_A$, $III_A$, $IV_A$, $I_B$, $II_B$, $III_B$, $IV_B$, $VI_B$ et VIII de la classification périodique des éléments. On citera à titre d'exemple, les cations alcalins, alcalino-terreux, les cations des terres rares, $Fe^{II}$, $Fe^{III}$, $Co^{II}$, $Co^{III}$, $Ni^{II}$, $Cu^{II}$, $Zn^{II}$, $Ag^+$.

D'autres agents structurants que ceux cités précédemment peuvent être utilisés, comme il est reconnu dans l'art antérieur, notamment des composés ayant des fonctions amine, cétone, acide par exemple des amino-alcools, des amino-acides, des polyalcools ou des amines tertiaires. Parmi les amines que l'on peut utiliser de manière préférée on peut citer $C_2H_5$-$NH_2$, $nC_3H_7$-$NH_2$, $nC_4H_9$-$NH_2$, $NH_2$-$(CH_2)_2$-$NH_2$, $NH_2$-$(CH_2)_3$-$NH_2$, $(CH_3)_2NH$, $(C_2H_5)_2NH$, $CH_3$-$NH$-$C_2H_5$, $CH_3$-$NH$-$C_3H_7$, $NH_2CH_2$-$NH$-$CH_3$, $NH_2$-$CH_2$-$NH$-$CH_2$-$NH_2$.

Parmi les sources d'éléments $Si^{IV}$ qui peuvent être utilisées on peut citer les silices sous forme d'hydrogels, d'aérogels, de suspensions colloïdales, les silices résultant de la précipitation de solutions de silicates solubles ou de l'hydrolyse d'esters siliciques comme $Si(OC_2H_5)_4$ ou des complexes comme $(NH_4)_2$ $SiF_6$, les silices préparées par des traitements d'extraction de composés naturels ou synthétiques comme les silicates d'aluminium, les aluminosilicates, les argiles. La silice peut aussi être introduite sous forme de silicates alcalins solubles ou de leur solution.

Parmi les sources de l'élément $B^{III}$ qui peuvent être utilisées on peut citer l'acide borique $H_3BO_3$ ou l'anhydride borique $B_2O_3$, mais des sels comme le borax, le tétraborate d'ammonium ou des molécules hydrolysables comme $BF_3$, $BCl_3$ et les esters de l'acide borique, comme le triéthylborate, conviennent également.

Parmi les sources de l'élément $Al^{III}$ qui peuvent être utilisées on peut citer les sels d'aluminium (sulfate, nitrate, chlorure, fluorure, acétate par exemple), les hydroxydes, les hydroxyoxydes et oxydes d'aluminium,

4

les aluminates, les esters comme l'ester tripropylique d'aluminium $Al(OC_3H_7)_3$.

Parmi les sources de l'élément $Ga^{III}$ qui peuvent être utilisées on peut citer les sels de gallium (sulfate, nitrate, chlorure, fluorure, acétate par exemple) les hydroxydes, les hydroxyoxydes et oxydes de gallium, les gallates et différents esters.

Parmi les sources de l'élément $Fe^{III}$ qui peuvent être utilisées on peut citer les sels de fer trivalent (sulfate, nitrate, chlorure, fluorure, acétate par exemple) les hydroxydes, hydroxyoxydes et oxydes ferriques.

Au lieu de partir de sources avec les différents éléments séparés on peut aussi utiliser des sources où l'élément $Si^{IV}$ est combiné à un ou plusieurs des éléments de la série $B^{III}$, $Al^{III}$, $Ga^{III}$ et $Fe^{III}$ ainsi que des sources où deux ou plusieurs éléments de cette dernière série sont associés. C'est par exemple le cas de gel de silice alumine, de verres borosilicatés, d'oxydes ou hydroxydes mixtes de gallium et d'aluminium.

Les différentes sources des éléments T des tétraèdres $TO_{4/2}$ de la charpente peuvent être engagées sous forme de solutions, de gels, de solides cristallins mais également sous forme d'agglomérats comme des extrudés ou des pastilles qui seront alors transformés en ferriérite déjà aggloméré.

Les cations ammonium dérivant des amines $(C, N)_{3-8}$ et de préférence $(C, N)_{3-5}$ nécessaires à la cristallisation de la ferriérite sont ajoutés au mélange réactionnel sous forme d'un de leurs sels comme par exemple le chlorhydrate ou le fluorhydrate de n-propylammonium ou de n-butyl-ammonium. On peut aussi ajouter une ou plusieurs amines $(C, N)_{3-8}$ qui seront ensuite transformées in situ en cations lors de l'ajustement du pH à une valeur inférieure à 10.

Les anions fluorures sont ajoutés sous forme d'acide fluorhydrique ou de fluorures de métaux alcalins, par exemple le fluorure de sodium, le fluorure d'ammonium $NH_4F$, le bifluorure d'ammonium $NH_4HF_2$, les fluorhydrates d'amines $(C, N)_{3-8}$ ou de composés hydrolysables pouvant libérer des anions fluorures dans l'eau, comme par exemple $SiF_4$ ou $(NH_4)_2SiF_6$. L'acide fluorhydrique, le fluorure d'ammonium ou le bifluorure d'ammonium sont des produits préférés car ils sont peu onéreux et ils permettent d'obtenir des ferriérites protonées par simple calcination de la zéolithe résultant de la synthèse.

Le pH du milieu réactionnel est inférieur à 10, avantageusement compris entre 3,5 et 10 et de manière plus préférée entre 4,5 et 9. Il peut être obtenu soit directement à partir de l'un ou de plusieurs des produits comme HF ou $NH_4HF_2$ entrant dans la composition du milieu réactionnel, soit par l'ajout audit milieu d'un acide, d'une base, d'un sel acide, d'un sel basique ou d'un mélange tampon complémentaire.

L'ajout de cristaux (germes) de ferriérite au mélange réactionnel dans une proportion ne dépassant pas 10 % en poids par rapport au poids de silice présente, facilite généralement la cristallisation.

Le chauffage du mélange réactionnel est fait de préférence dans un autoclave revêtu intérieurement de polytétrafluoroéthylène (PTFE). Suivant la composition, l'ajout de germes, la température et l'agitation ou non, la durée du chauffage se situe généralement entre 12 et 350 heures. Lorsque la cristallisation est achevée, le solide obtenu est filtré et lavé à l'eau désionisée.

L'ajout de germes et l'agitation ont également une influence sur la taille des cristaux de ferriérite formés. En utilisant ces deux facteurs on peut faire varier la taille des cristaux entre quelques fractions de micromètres et quelques centaines de micromètres. Les cristaux ont généralement une forme de plaquettes.

Lors de l'étape de calcination des cristaux, on chauffe ceux-ci en présence d'un gaz, de préférence sec et contenant, lorsque les cristaux contiennent des cations décomposables autres que $NH_4^+$, de préférence de l'oxygène moléculaire. La température de calcination est supérieure à 400°C et de préférence comprise entre 500 et 600°C de manière à décomposer les cations $NH_4^+$ et/ou organiques contenus dans le solide.

Les produits obtenus par le procédé selon l'invention sont identifiés de manière commode à partir de leur diagramme de diffraction des rayons X. Il peut être obtenu à l'aide d'un diffractomètre en utilisant la méthode des poudres avec le rayonnement du cuivre. A partir de la position des pics de diffraction, représentée par l'angle $2\theta$, on calcule, par la relation de Bragg, les équidistances réticulaires $\Delta d_{hkl}$ caractéristiques de l'échantillon. L'estimation de l'erreur de mesure $\Delta d_{hkl}$ se calcule en fonction de l'erreur $\Delta 2\theta$ affecté à la mesure de $2\theta$ par la relation de Bragg. Une erreur $\Delta 2\theta$ égale à $\pm 0,2°$ est couramment admise. L'intensité relative $I/Io$, I étant l'intensité d'une raie donnée et Io l'intensité de la raie la plus forte, affectée à chaque valeur de $d_{hkl}$ est estimée à partir de la hauteur du pic de diffraction correspondant. Le tableau I ci-après représente les diagrammes de diffraction des rayons X caractéristiques d'une ferriérite avant calcination et une ferriérite calcinée à 750°C sous argon, les deux produits étant obtenus selon l'invention. Dans les colonnes des $d_{hkl}$ on a représenté les valeurs extrêmes que peuvent prendre les différentes équidistances $d_{hkl}$. Les valeurs dépendent du rapport $Si^{IV}/T^{III}$, de la nature de $T^{III}$ et de la nature des cations de compensation, chacune des valeurs indiquée dans le tableau doit encore être affectée de l'erreur de mesure $\Delta d_{hkl}$. Pour caractériser les intensités relatives $I/Io$, on a utilisé une échelle de symbole souvent utilisée : FF = très fort, F = fort, mF = moyen à fort, m = moyen, mf = moyen à faible, f = faible, ff = très faible, fff = très très faible. Ces intensités relatives dépendent également en partie de la

composition des ferriérites.

## TABLEAU 1 : Diagrammes de diffraction RX

| FERRIERITE NON CALCINEE SELON L'INVENTION | | FERRIERITE CALCINEE SELON L'INVENTION | |
|---|---|---|---|
| $d_{hkl}$ (Å) | I/Io | $d_{hkl}$ (Å) | I/Io |
| 11,10 - 11,40 | ff | 11,10 - 11,40 | ff |
| 9,20 - 9,50 | FF | 9,20 - 9,50 | FF |
| 7,55 - 7,80 | ff(f) | 7,55 - 7,80 | ff(f) |
| 6,95 - 7,20 | f | 6,95 - 7,20 | m |
| 6,85 - 7,10 | fm | 6,85 - 7,10 | m |
| 6,50 - 6,70 | f | 6,45 - 6,65 | fm |
| 6,05 - 6,25 | ff | | |
| 5,65 - 5,85 | f | 5,65 - 5,85 | f |
| 5,60 - 5,80 | f | 5,60 - 5,80 | fm |
| 4,85 - 5,05 | ff | | |
| 4,72 - 4,88 | ff | 4,70 - 4,85 | f |
| 4,62 - 4,76 | ff | | |
| 3,90 - 4,02 | m | 3,90 - 4,02 | m |
| 3,84 - 3,95 | m | 3,84 - 3,95 | m |
| 3,78 - 3,89 | fm | 3,78 - 3,89 | fm |
| 3,72 - 3,83 | m | 3,72 - 3,83 | m |
| 3,54 - 3,70 | fm | 3,59 - 3,70 | fm |
| 3,48 - 3,58 | m | 3,48 - 3,58 | m |
| 3,40 - 3,50 | m | 3,40 - 3,50 | m |
| 3,31 - 3,41 | f | 3,31 - 3,41 | f |
| 3,26 - 3,35 | f | 3,26 - 3,35 | f |
| 3,02 - 3,16 | f (large) | 3,08 - 3,16 | fm |
| 2,99 - 3,07 | f | 2,99 - 3,07 | f |
| 2,90 - 2,98 | ff | 2,90 - 2,98 | f |
| 2,83 - 2,91 | ff | 2,84 - 2,92 | f |
| 2,79 - 2,86 | ff | 2,79 - 2,86 | ff |
| 2,66 - 2,74 | ff | 2,66 - 2,74 | f |
| 2,60 - 2,67 | ff | 2,60 - 2,67 | f |

La ferriérite de la présente invention peut être utilisée comme catalyseur soit pure soit associée à une matrice comprenant des composés choisis dans le groupe formé par l'alumine, la silice, la magnésie, la zircone, l'oxyde de titane, l'oxyde de bore,une argile et toute combinaison d'au moins deux composés précités. La teneur en ferriérite du catalyseur sera au moins égale à 3 % poids et de préférence au moins égale à 20 % et la teneur en matrice sera au plus égale à 97% en poids et de préférence au plus égale à 80 %. Eventuellement le catalyseur peut comprendre une fonction hydrogénante-déshydrogénante apportée par au moins un métal choisi de préférence parmi les groupes IB et VIII ou apportée par au moins un sulfure de métal choisi parmi les groupes IB et VIII.

Les ferriérites préparées selon l'invention sont utilisées à titre de catalyseurs ou support de catalyseur pur ou en mélange dans des réactions de conversion d'hydrocarbures et plus particulièrement dans la réaction d'oligomérisation des oléfines.

EXEMPLE 1. Préparation en milieu fluorure d'une ferriérite de rapport molaire $SiO_2/Al_2O_3$ égal à 40.

On utilise comme source de silice et d'alumine un alumino-silicate de sodium commercialisé sous le nom de Tixolex 28 (marque déposée) et préalablement traité par une solution aqueuse acide afin d'augmenter le rapport Si/Al et d'éliminer les cations $Na^+$. Pour cela on traite 350 g de Tixolex 28 par 3,5 litres de $HNO_3$(M/2) à température ambiante pendant 3 heures sous agitation. Le solide est ensuite filtré, lavé à l'eau puis séché à 60°C, et conservé sur humidificateur. Dans l'exemple décrit ici et dans ceux qui suivent ce produit est appelé "Tixolex traité $HNO_3$" et il a comme composition $SiO_2$ 77,5 %, $Al_2O_3$ 5,2 %, $Na_2O$ 0,15 %, $H_2O$ 16,5 % (rapport $SiO_2/Al_2O_3 \approx 25$).

9,24 g de Tixolex traité $HNO_3$ sont dispersés sous agitation dans une solution contenant 23,9 g $H_2O$, 9,73 g HF à 40 % et 9,2 ml de n-propylamine. Ce mélange réactionnel ayant un pH de 7 est ensuite chauffé 12 jours à 170°C dans un autoclave revêtu de polytétrafluoroéthylène (PTFE). Après refroidissement les cristaux présents dans une eau mère à pH 7,5 sont filtrés et lavés à l'eau puis séchés. Leur taille varie de 20 à l25 micromètres et le rapport molaire $SiO_2/Al_2O_3$ déterminé par analyse chimique après calcination sous mélange 20 % air dans 80 % $N_2$ à 550°C est égal à 40. Les cristaux recueillis ont un diagramme de diffraction X typique de la ferriérite (voir tableau 1), aucune autre phase n'est détectée. La teneur en fluor sur le solide brut de synthèse est égale à 0.78 % poids.

EXEMPLE 2. Préparation en milieu fluorure d'une ferriérite de rapport molaire $SiO_2/Al_2O_3$, égal à 30.

On incorpore sous agitation 3,36 g de Tixolex traité $HNO_3$ préparé selon l'exemple 1 dans une solution contenant 20,6 g $H_2O$, 1,77 g HF à 40 % et 3,33 ml de n-propylamine. Après chauffage en autoclave revêtu par du PTFE à 200°C, on obtient au bout de 3 jours dans une eau mère de PH égal à 7 (mesuré après refroidissement) des cristaux de 40µm environ qui sont filtrés, lavés et séchés. Ces cristaux ont un diagramme de diffraction X typique de la ferriérite, aucune autre phase n'étant détectée. L'analyse chimique réalisée après calcination sous mélange 20 % air dans 80 % $N_2$ à 550°C donne un rapport molaire $SiO_2/Al_2O_3$ voisin de 30. La teneur en fluor des cristaux bruts de synthèse est égale à 0,65 % poids.

EXEMPLE 3. (comparatif) Influence néfaste de rapports $F^-/Si$ et cation organique/$F^-$ mal adaptés.

Cet exemple montre que des rapports molaires $F^-/Si$ et n-propylamine/Si trop faibles peuvent conduire à une cristallisation incomplète. En partant des mêmes réactifs que ceux de l'exemple 2 on réalise un mélange réactionnel caractérisé par la valeur 0,05 des deux rapports molaires ci-dessus. Pour cela on mélange 4,2 g de Tixolex traité $HNO_3$ (voir l'exemple 1) avec 0,62 ml n-propylamine, 0,33 ml HF à 40 % et 30 ml $H_2O$ et 0,06 g de cristaux de ferriérite servant de germes. Le mélange chauffé en autoclave revêtu de PTFE à 200°C, pendant 4 jours, conduit après filtration et lavage, à un solide ne contenant que 15 % de ferriérite (détermination par diffraction X).

EXEMPLE 4. (comparatif) Influence néfaste d'un pH trop élevé.

Cet exemple montre qu'un mélange réactionnel ayant un pH trop élevé peut conduire à une ferriérite contenant des impuretés cristallisées comme la cristobalite.

On réalise un mélange contenant 5,04 de Tixolex traité $HNO_3$ (cf exemple 1), 5 ml de n-propylamine, 1,71 g de $NH_4HF_2$ et 30 g de $H_2O$. Le mélange ayant un pH de 10,2 est chauffé à 200°C, pendant 4 jours dans un autoclave revêtu de PTFE. Après refroidissement le pH est de 10,8 et le solide obtenu après filtration et lavage est un mélange de ferriérite (40 %) et de cristobalite (60 %) (détermination par diffraction X).

EXEMPLE 5. Préparation de ferriérites selon l'invention à partir de cations organiques différents.

On utilise comme source de silice et d'alumine un alumino-silicate de sodium commercialisé sous le nom de Tixolex 28 et préalablement échangé contre des cations $NH_4^+$ de manière à éliminer les cations $Na^+$. Pour cela on traite 250 g de Tixolex 28 par 2,5 litres de $NH_4 NO_3$ (M) pendant 16 heures 60°C. Après filtration et lavage à l'eau ce traitement est répété deux fois. Après le dernier lavage le produit est séché à 60°C puis conservé sur humidificateur. Sa composition est $SiO_2$ 73,5 %, $Al_2O_3$ 8,7 %, $Na_2O$ 0,02 %, $(NH_4)_2O$ + $H_2O$ 17,8 %, le rapport molaire $SiO_2/Al_2O_3$ étant de 14,4. Cette source de silice et d'alumine est appelée : Tixolex échangé $NH_4^+$.

Les quatre essais (5a, 5b, 5c et 5d) de cet exemple sont réalisés avec le Tixolex échangé $NH_4^+$ et quatre cations alkylammonium différents. les compositions molaires du mélange réactionnel ramenées à 1 mole de $SiO_2$ contenue dans le Tixolex échangé $NH_4^+$, sont indiquées, ainsi que les conditions de synthèse, dans le tableau 2.

Pour les quatre essais, on obtient après réaction à l'autoclave et séparation des eaux mères, des cristaux de ferriérite 16 dont les spectres de diffraction entrent dans les spécifications du tableau 1, (aucune autre phase n'est détectée par diffraction X ou microscopie). Les eaux mères ont des pH compris entre 6,5 et 9,0. Les teneurs en fluor des solides bruts de synthèse sont respectivement égale à 0,54, 0,30, 0,40 et 0,60 % poids pour les échantillons 5a, 5b, 5c et 5d. Après calcination à 550°C, l'analyse chimique révèle pour les échantillons 5a, 5b, 5c et 5d des rapports $SiO_2/Al_2O_3$ molaires respectivement égaux à 28, 22, 32 et 22.

### TABLEAU 2 : Composition du milieu de synthèse des solides préparés selon l'exemple 5.

| Composition molaire | | ESSAI 5A | ESSAI 5b | ESSAI 5c | 5d |
|---|---|---|---|---|---|
| | $SiO_2$ (dans Tixolex échangé $NH_4^+$) | 1 | 1 | 1 | 1 |
| | $Al_2O_3$ (dans Tixolex échangé $NH_4^+$) | 0,07 | 0,07 | 0,07 | 0,07 |
| | Alkylamine | 2 *<br>Ethylamine | 1<br>n-propyl-amine | 3<br>n-butyl-amine | 1<br>1,3-diamino propane |
| | HF | 2 | 1 | 3 | 2,5 |
| | $H_2O$ | 10,5 | 30 | 10 | 10 |
| Masse de Tixolex échangé $NH_4^+$ engagé | | 3,6 g | 9,9 g | 3,6 g | 3,6 g |
| Masse de germes de ferriérite | | – | 0,13 g | – | 0,13 g |
| Température °C | | 170 | 170 | 170 | 170 |
| Durée | | 14 jours | 17 jours | 15 jours | 10 jours |

* Ajoutée sous forme d'une solution d'éthylamine à 70 % dans l'eau.

EXEMPLE 6 : Préparation selon l'invention de ferriérites de rapports $SiO_2/Al_2O_3$ supérieurs à 70.

Les deux essais de l'exemple 6 montrent qu'on peut synthétiser par cette méthode des ferriérites dont le rapport $SiO_2/Al_2O_3$ atteint et dépasse même la valeur 100.

L'essai 6a a été effectué avec un mélange réactionnel contenant 7 g de silice-alumine (94,65 % $SiO_2$, 2,5 % $Al_2O_3$ et 2,7 % $H_2O$), 9,17 ml de n-propylamine, 4,86 ml HF à 40 % dans l'eau, 56,5 ml d'eau et 0,13 g de ferriérite comme germe. Après réaction à l'autoclave pendant 15 jours à 170°C, les cristaux obtenus sont séparés des eaux-mères, dont le pH est voisin de 6,5, par filtration et lavage. Ces cristaux sont comme le montre la diffraction X constitués uniquement de ferriérite. Aucune autre phase n'est détectée soit par diffraction X soit par microscopie. Les cristaux sont calcinés à 550°C puis analysés chimiquement : leur rapport $SiO_2/AL_2O_3$ est égal à 75. La teneur en fluor des cristaux bruts de synthèse est égale à 1,25 % poids.

L'essai 6b a été effectué avec un mélange réactionnel contenant 3,6 g de silice Merck précipitée (rapport $SiO_2/Al_2O_3$ voisin de 560 dû à la présence d'alumine comme impureté), 7,3 g de n-butylamine, 4,42 ml HF à 40 % dans l'eau, 6 g d'eau et 0,3 g de ferriérite servant de germes. Après réaction à l'autoclave pendant 15 jours à 170°C, on sépare un solide par filtration et lavage à l'eau. Le solide contient environ 80 % de cristaux de ferriérite à côté de 20 % d'amorphe qu'on peut éliminer facilement par décantation après sonication. Les cristaux de ferriérite ainsi purifiés ont un rapport $SiO_2/Al_2O_3$ de 250.

EXEMPLE 7 : Préparation selon l'invention de ferriérites contenant comme éléments trivalents (Al, Ga), (Al, Fe) et (Al, B).

Les quatre essais de l'exemple 7 montrent qu'on peut synthétiser par cette méthode des ferriérites contenant d'autres êléments trivalents que l'aluminium comme par exemple le fer, le gallium et le bore.

Le premier mélange réactionnel (essai 7a) contient 3,36 g de Tixolex traité $HNO_3$ (cf exemple 1) dispersé dans une solution préparée avec 20,6 g d'eau, 1,77 g HF à 40 % dans l'eau, 3,33 ml de n-propylamine et 0,7 g $GaCl_3$.

Dans le deuxième mélange réactionnel (essai 7b), on remplace $GaCl_3$ par 1,08 g $Fe_2O_3$, $6H_2O$ et 0,12 g de ferriérite servant de germes.

Dans le troisième mélange réactionnel (essai 7c), on remplace les 0,7g de $GaCl_3$ du mélange 7a par 0,07g du même composé.

Dans le quatrième mélange réactionnel (essai 7d), on remplace le gallium par 0,6g de $H_3BO_3$.

Le mélange réactionnel de l'essai 7a est chauffé en autoclave à 210°C pendant 2 jours. Après refroidissement, le solide, filtré et lavé, est constitué de cristaux de ferriérite caractérisé par les rapports $SiO_2/Al_2O_3$ et $SiO_2/Ga_2O_3$ respectivement égaux à 28 et 35. Le pH des eaux-mères mesuré après refroidissement est voisin de 8. La teneur en fluor du solide brut de synthèse est égale à 0.65% poids. Le spectre de diffraction X est comparable à celui présenté dans le tableau 1.

Le mélange réactionnel de l'essai 7b est chauffé en autoclave à 170°C, pendant 12 jours. Après refroidissement, le pH est voisin de 7 et le solide, filtré et lavé, est constitué de cristaux de ferriérite avec un peu d'amorphe. Après séparation de la phase amorphe par décantation (sonication) l'analyse chimique montre que les cristaux de ferriérite contiennent 2,2 % $Al_2O_3$ et 1,75 % de $Fe_2O_3$. La teneur en fluor des cristaux bruts de synthèse est égale à 0.68 % poids et le spectre de diffraction X est sensiblement identique à celui présenté dans le tableau 1.

Les mélanges réactionnels des essais 7c et 7d sont traités comme dans l'essai 7a. Les cristaux de ferrierite obtenus dans les deux cas donnent un spectre de diffraction du même type que celui présenté dans le tableau I. L'analyse chimique effectuée sur les produits avant calcination donne les résultats suivants :

Essai 7c $SiO_2/Al_2O_3$ = 26 $SiO_2/Ga_2O_3$ = 380 % F (poids) = 0,90
Essai 7d $SiO_2/Al_2O_3$ = 28 $SiO_2/B_2O_3$ = 45 % F (poids = 0,70

EXEMPLE 8: Préparation selon l'invention de ferriérites riches en silicium et contenant comme élément trivalent du gallium.

Cet exemple montre qu'il est possible de préparer des ferriérites où le silicium est substitué par d'autres éléments trivalents que l'aluminium.

25 ml de $Si(OC_2H_5)_4$, 2,1 g de $GaCl_3$ et 50 ml $H_2O$ sont portés à reflux pendant 3 heures. Après précipitation du gel gallosilicaté, ce dernier est séché à 80°C, puis finement broyé. L'analyse chimique du gel indique un rapport molaire $SiO_2/Ga_2O_3$ de 20 avec ce gel. On réalise ensuite un mélange dont la composition molaire est la suivante : 1 $SiO_2$, 0,05 $Ga_2O_3$, 2 n-propylamine, 2 HF, 20 $H_2O$. La fraction molaire engagée est égale à 0,1 et le mélange contient 0,15 g de cristaux de ferriérite comme germes. Le pH initial est égal à 7. Le mélange placé dans un autoclave chemisé par du PTFE est porté à 200°C, pendant 6 jours. Pendant le chauffage l'autoclave cylindrique tourne, autour d'un axe perpendiculaire à l'axe de cylindre, une vitesse de 15 tours/minute. Après refroidissement le pH mesuré dans le milieu réactionnel est de 7,5. Les cristaux séparés par filtration et lavage sont constitués de ferriérite possédant un rapport molaire $SiO_2/Ga_2O_3$ de 24. La teneur en fluor du solide brut de synthèse est égale à 0,55 % poids et le

EP 0 269 503 B1

spectre de diffraction X est sensiblement le même que celui présenté dans le tableau 1.

EXEMPLE 9. Préparation selon l'invention de ferriérites servant de catalyseurs pour l'oligomérisation des oléfines.

La ferriérite obtenue par l'essai 6a de l'exemple 6 dont le rapport molaire $SiO_2/Al_2O_3$ est égal 75 est calcinée à 550°C pendant 4 heures sous un mélange 20 % air + 80 % azote (débit total 10 litres $h^{-1}g^{-1}$).

A l'issue de ce traitement le solide est référencé Fer1, sa teneur en fluor est égale à 1,25 % poids.

La ferriérite forme H Fer1 est traitée dans une solution de $NH_4OH$ de normalité 0.2N dans un autoclave à 160°C pendant 4 heures. Après ce traitement la zéolithe est lavée sur filtre puis calcinée à 550°C pendant 4 heures sous un mélange 20 % air + 80 % azote (débit total 10 litres $h^{-1}g^{-1}$).

A l'issue de ce traitement le solide est référencé Fer2, sa teneur en fluor est inférieure à 0,01 % en poids.

EXEMPLE 10 : Test en oligomérisation du propène des ferriérites Fer1 et Fer2.

Un test d'oligomérisation du propène a été réalisé sur 10 g de ferriérite (Fer1, Fer2) placé dans un réacteur de 65 cm$^3$ de capacité. Le catalyseur était placé entre deux lits d'alumine alpha, l'alumine alpha se comporte comme un solide inerte pour la réaction d'oligomérisation.

Le propène a été injecté dans les conditions suivantes :
- Température : 330°C
- Pression : 4 MPa.

Dans ces conditions opératoires le taux de transformation du propène est respectivement de 89 % et 75 % pour les catalyseurs Fer1 et Fer2.

Les produits liquides soutirés après transformation du propène non transformé pour les deux catalyseurs présentaient les caractéristiques suivantes :

|  | Fer1 | Fer2 |
|---|---|---|
| Densité à 20°C | 0,788 | 0,771 |
| Indice de brome | 74 | 86 |
| Distillation ASTM | | |
|     Point initial °C : | 78°C | 58°C |
|     10 % vol : | 143°C | 123°C |
|     30 % vol : | 167°C | 149°C |
|     50 % vol : | 210°C | 195°C |
|     70 % vol : | 253°C | 237°C |
|     90 % vol : | 298°C | 283°C |
|     95 % vol : | 313°C | 299°C |
|     Point final°C | 322°C | 310°C. |

Les répartitions par nombre d'atomes de carbone déterminées par spectrométrie de masse sont les suivantes :

|  | Fer1 | Fer2 |
|---|---|---|
| C6 | 8,5 % | 13,9 % |
| C7 | 3,7 % | 4,8 % |
| C8 | 4,9 % | 5,7 % |
| C9 | 18,0 % | 20,8 % |
| C10 | 6,1 % | 6,9 % |
| C11 | 7,3 % | 7,5 % |
| C12 | 20,4 % | 16,9 % |
| C13 | 5,2 % | 4,8 % |
| C14 | 4,7 % | 4,2 % |
| C15 | 11,8 % | 7,9 % |
| C16 | 4,1 % | 3,1 % |
| C17 | 2,8 % | 1,7 % |
| $C18^{+}$ | 2,5 % | 1,8 % |
|  | 100,0 % | 100,0 % |

Il apparait donc au vu des résultats catalytiques que la présence de fluor en plus grande quantité dans Fer1 que dans Fer2 permet d'améliorer les performances en oligomérisation des oléfines.

EXEMPLE 11 : Test en oligomérisation des ferriérites au gallium, bore aluminium et fer.

On a réalisé le test d'oligomérisation du propène selon l'exemple 10 à la différence près que l'on a substitué la ferriérite Fer 1 de l'exemple 10 successivement par la ferriérite (Al, Ga) puis par la ferriérite (Al, Fe), ensuite par la ferriérite (Al, B) de l'exemple 7 et enfin par la ferriérite au gallium de l'exemple 8. On a obtenu sensiblement des résultats aussi satisfaisants que ceux obtenus avec la ferriérite de l'exemple 10 aussi bien au niveau de la conversion que de la sélectivité.

**Revendications**

1. Une ferriérite synthétique cristalline caractérisée par :
   a) la formule suivante :

   $M_{x/n}(T^{III}O_2)x(Si^{IV}O_2)_{36-x}$

   où $T^{III}$ représente au moins un élément choisi dans le groupe du bore ($B^{III}$), de l'aluminium ($Al^{III}$), du gallium ($Ga^{III}$) et du fer ($Fe^{III}$)
   $x$ est compris entre 0.05 et 6,6
   $M$ est un cation de compensation
   $n$ est la valence de $M$.
   b) une teneur en fluor après synthèse comprise entre 0,01 et 1,6 % en poids,
   c) un rapport molaire $Si^{IV}/T^{III}$ au moins égal à 4,75 ; et
   d) un diagramme de diffraction X représenté dans le tableau 1 ci- après :

TABLEAU 1 : Diagrammes de diffraction RX

| FERRIERITE CALCINEE SELON L'INVENTION | |
| --- | --- |
| $d_{hkl}$ (Å) | I/Io |
| 11,10 - 11,40 | ff |
| 9,20 - 9,50 | FF |
| 7,55 - 7,80 | ff(f) |
| 6,95 - 7,20 | m |
| 6,85 - 7,10 | m |
| 6,45 - 6,65 | fm |
| 5,65 - 5,85 | f |
| 5,60 - 5,80 | fm |
| 4,70 - 4,85 | f |
| 3,90 - 4,02 | m |
| 3,84 - 3,95 | m |
| 3,78 - 3,89 | fm |
| 3,72 - 3,83 | m |
| 3,59 - 3,70 | fm |
| 3,48 - 3,58 | m |
| 3,40 - 3,50 | m |
| 3,31 - 3,41 | f |
| 3,26 - 3,35 | f |
| 3,08 - 3,16 | fm |
| 2,99 - 3,07 | f |
| 2,90 - 2,98 | f |
| 2,84 - 2,92 | f |
| 2,79 - 2,86 | ff |
| 2,66 - 2,74 | f |
| 2,60 - 2,67 | f |

FF = très fort, m = moyen, fm - moyen à faible, f = faible

ff = très faible, fff = très très faible

2. Une ferriérite selon la revendication 1 caractérisée en ce que le rapport molaire $Si^{IV}/T^{III}$ est compris entre 7 et 500.

3. Catalyseur renfermant en poids :

a) au moins 3 % et de préférence au moins 20 % en poids de ferriérite selon l'une quelconque des revendications 1 et 2

b) au plus 97 % et de préférence au plus 80 % en poids d'une matrice choisie dans le groupe formé par l'alumine, la silice, la magnésie, la zircone, l'oxyde de titane, l'oxyde de bore, une argile et toute combinaison d'au moins deux des composés précités.

4. Procédé de préparation d'une ferriérite synthétique cristalline selon l'une des revendications 1 et 2, caractérisée en ce que :

a) on prépare un mélange réactionnel en solution aqueuse à un pH inférieur à 10, avantageusement compris entre 3,5 et 9, comprenant au moins une source d'oxyde de silicium, une source d'au moins un oxyde de métal trivalent T choisi dans le groupe constitué par le bore, le gallium, l'aluminium et le fer, une source d'ion fluorure $F^-$ et au moins un agent structurant fournissant des cations organiques contenant de l'azote choisi dans le groupe formé par les monoamines, les diamines, les triamines aliphatiques primaires et secondaires et les cations ammonium dérivant par protonation des dites mono-, di-, triamines, ledit agent ayant un nombre total d'atomes de carbone et d'azote de 3 à 8, ledit mélange ayant une composition, en termes de rapports molaires, comprise dans les intervalles de valeurs suivants :

$Si^{IV}/\Sigma T^{III}$ 2-400 ($T^{III}$ = B, Al, Ga, Fe)

$F^-/Si^{IV}$ 0,1-3

Structurant organique/$Si^{IV}$ 0,1-4

$H_2O/Si^{IV}$ 4-200

b) on maintient ledit mélange à une température de chauffage au plus égale à 270°C, de façon à obtenir des cristaux, et

c) on calcine lesdits cristaux à une température supérieure à 400°C.

5. Procédé selon la revendication 4 dans lequel ledit mélange en solution aqueuse a un pH et une composition en termes de rapports molaires compris dans les intervalles suivants :

PH 4,5-9

$Si^{IV}/\Sigma T^{III}$ 4-150 ($T^{III}$ = B, Al, Ga, Fe)

$F^-/si^{IV}$ 0,25-2,5

Structurant organique/$Si^{IV}$ 0,25-2,5

$H_2O/Si^{IV}$ 6-80.

6. Procédé selon l'une des revendications 4 et 5 dans lequel la température de chauffage dudit mélange réactionnel est maintenue entre 120 et 260°C.

7. Procédé selon l'une des revendications 4 à 6 dans lequel on calcine lesdits cristaux à une température comprise entre environ 500 et 600°C.

8. Procédé selon l'une des revendications 4 à 7 dans lequel lesdits cristaux sont calcinés en présence d'un gaz comprenant de l'oxygène moléculaire.

9. Procédé selon l'une des revendications 4 à 8 dans lequel on remplace au moins une partie des cations d'origine par échange ionique par un cation ou mélange de cations choisis parmi les groupes IIA, IIIA, IVA, IB, IIB, IIIB, IVB, VIB, et VIII du tableau périodique des éléments.

10. Utilisation d'une ferriérite ou d'un catalyseur contenant une ferriérite selon les revendications 1 à 3 ou préparée selon l'une des revendications 4 à 9 dans un procédé d'oligomérisation des oléfines.

**Claims**

1. A crystaline synthetic ferrierite characterized by :

a) the following approximate formula :

$M_{x/n}(T^{III}O_2)_x(Si^{IV}O_2)_{36-x}$

where $T^{III}$ represents at least one element selected from the group of boron ($B^{III}$), aluminum ($Al^{III}$), gallium ($Ga^{III}$) and iron ($Fe^{III}$)

x ranges from 0.05 to 6.6

M is a compensation cation

n is the valence of M.

b) a fluorine content after synthesis ranging from about 0.01 to 1.6 % by weight,

c) a $Si^{IV}/T^{III}$ molar ratio of at least about 4.75 ; and

d) a X-ray diffraction diagram shown in Table 1 hereinafter.

| ROASTED FERRIERITE ACCORDING TO THE INVENTION | | | | |
|---|---|---|---|---|
| $d_{hkl}$ (Å) | I/Io | | | |
| 11.10 – 11.40 | vl | | 3.72 – 3.83 | m |
| 9.20 – 9.50 | vs | | 3.59 – 3.70 | lm |
| 7.55 – 7.80 | vvl | | 3.48 – 3.58 | m |
| 6.95 – 7.20 | m | | 3.40 – 3.50 | m |
| 6.85 – 7.10 | m | | 3.31 – 3.41 | l |
| 6.45 – 6.65 | lm | | 3.26 – 3.35 | l |
| | | | 3.08 – 3.16 | lm |
| 5.65 – 5.85 | l | | 2.99 – 3.07 | l |
| 5.60 – 5.80 | lm | | 2.90 – 2.98 | l |
| | | | 2.84 – 2.92 | l |
| 4.70 – 4.85 | l | | 2.79 – 2.86 | vl |
| | | | 2.66 – 2.74 | l |
| 3.90 – 4.02 | m | | 2.60 – 2.67 | l |
| 3.84 – 3.95 | m | | | |
| 3.78 – 3.89 | lm | | | |

vs = very strong, m = medium, lm = medium to low, l = low, vl = very low, vvl = very very low.

2. A ferrierite according to claim 1, characterized in that the $Si^{IV}/T^{III}$ molar ratio ranges from about 7 to about 500.

3. A catalyst containing by weight :

a) at least 3% and preferably 20% by weight of ferrierite according to any one of claims 1 and 2,

b) at most 97% and preferably at most 80% by weight of a matrix selected from the group consisting of alumina, silica, magnesia, zirconia, titanium oxide, boron oxide, a clay or any combination of at least two of the above-mentioned compounds.

4. A process for manufacturing a crystalline synthetic ferrierite according to one of claims 1 and 2, characterized by the steps of :

a) preparing a reaction mixture in agueous solution at a pH lower than about 10, comprising at least one source of silicium oxide, one source of at least one oxide of trivalent metal T selected from the group consisting of boron, gallium, aluminum and iron, one source of fluorine ion $F^-$ and at least one

16

structurizing agent supplying nitrogen-containing organic cations, selected from the group formed of primary and secondary aliphatic monoamines, diamines, triamines and ammonium cations deriving by protonation from said mono-, di-, triamines, said agent having a total number of carbon and nitrogen atoms from 3 to 8, said mixture having a composition, in terms of molar ratios, ranging within the following intervals :

siIV/$\Sigma$TIII 2-400 (TIII = B, Al, Ga, Fe)

F$^-$/si$^{IV}$ 0.1-3

Organic structurizing agent/Si$^{IV}$ 0.1-4

$H_2O$/Si$^{IV}$ 4-200

b) maintaining said mixture at a heating temperature of at most 270°C, so as to obtain crystals, and

c) roasting said crystals at a temperature higher than 400°C.

5. A process according to claim 4, wherein said mixture in aqueous solution has a pH and a composition, in terms of molar ratios, ranging within the following intervals :

PH 4.5-9

Si$^{IV}$/$\Sigma$T$^{III}$ 4-150 (T$^{III}$ = B, Al, Ga, Fe)

Organic structurizing agent 0.25-2.5

$H_2O$/Si$^{IV}$ 6-80.

6. A process according to one of claims 4 and 5, wherein the heating temperature of said reaction mixture is maintained within the range of 120-260°C.

7. A process according to one of claims 4 to 6, wherein said crystals are roasted at a temperature within the range of about 500-600°C.

8. A process according to one of claims 4 to 7, wherein said crystals are roasted in the presence of a molecular oxygen-containing gas.

9. A process according to one of claims 4 to 8, wherein at least a part of the original cations is replaced, by ion exchange, with a cation or mixture of cations selected from groups IIA, IIIA, IVA, IB, IIB, IIIB, IVB, VIB, and VIII of the periodic classification of elements.

10. The use of a ferrierite or of a ferrierite-containing catalyst according to one of claims 1 to 3 or prepared according to one of claims 4 to 9, in a process for olefin oligomerization.

## Patentansprüche

1. Synthetischer kristalliner Ferrierit,
gekennzeichnet durch,
a) die folgende Formel:

$$M_{x/n}(T^{III}O_2)_x(Si^{IV}O_2)_{36-x}$$

wobei T$^{III}$ mindestens ein Element repräsentiert, das aus der Gruppe von Bor (B$^{III}$ ), Aluminium (Al$^{III}$ ), Gallium (Ga$^{III}$ ) und Eisen (Fe$^{III}$ ) gewählt ist, wobei
x zwischen 0,05 und 6,6 liegt
M ein Kompensationskation ist
n die Valenz von M ist
b) einen Gehalt an Fluor nach der Synthese zwischen 0,01 und 1,6 Gewichtsprozent,
c) ein Molverhältnis Si $^{IV}$/T $^{III}$ ,das mindestens gleich 4,75 ist; und
d) ein Röntgendiffraktionsdiagramm, das in der folgenden Tabelle 1 dargestellt ist:

17

Tabelle 1: Röntgendiffraktionsdiagramm   RX

| Kalzinierter Ferrierit gemäß der Erfindung | |
|---|---|
| $d_{hkl}$ (Å) | I/Io |
| 11,10 - 11,40 | ff |
| 9,20 - 9,50 | FF |
| 7,55 - 7,80 | ff(f) |
| 6,95 - 7,20 | m |
| 6,85 - 7,10 | m |
| 6,45 - 6,65 | fm |
| 5,65 - 5,85 | f |
| 5,60 - 5,80 | fm |
| 4,70 - 4,85 | f |
| 3,90 - 4,02 | m |
| 3,84 - 3,95 | m |
| 3,78 - 3,89 | fm |
| 3,72 - 3,83 | m |
| 3,59 - 3,70 | fm |
| 3,48 - 3,58 | m |
| 3,40 - 3,50 | m |
| 3,31 - 3,41 | f |
| 3,26 - 3,35 | f |
| 3,08 - 3,16 | fm |
| 2,99 - 3,07 | f |
| 2,90 - 2,98 | f |
| 2,84 - 2,92 | f |
| 2,79 - 2,86 | ff |
| 2,66 - 2,74 | f |
| 2,60 - 2,67 | f |

FF= sehr stark, m = mittel, fm = mittel bis schwach, f = schwach, ff = sehr schwach, fff = sehr sehr schwach

2. Ferrierit nach Anspruch 1,
dadurch gekennzeichnet,
daß das Molverhältnis si$^{IV}$/T$^{III}$ zwischen 7 und 500 liegt.

18

3. Katalysator, der folgende Gewichtsbereiche umfasst:

a) mindestens 3 Gewichtsprozent und vorzugsweise 20 Gewichtsprozent Ferrierit nach einem der Ansprüche 1 und 2,

b) mehr als 97 Gewichtsprozent und vorzugweise mehr als 80 Gewichtsprozent einer Matrix, aus der Gruppe, die aus Alumiumoxid, Siliziumoxid, Magnesiumoxid, Zirkonoxid, Titanoxid, Boroxid, einem Tonmineral und jeder Kombination von mindestens zwei der aufgeführten Verbindungen besteht.

4. Verfahren zur Herstellung eines synthetischen kristallinen Ferrierits nach einem der Ansprüche 1 und 2, dadurch gekennzeichnet,

a) daß man eine Reaktionsmischung in wässriger Lösung bei einem pH unterhalb von 10, vorzugsweise zwischen 3,5 und 9, herstellt, die mindestens eine Quelle für Siliziumoxid und mindestens eine Quelle für ein trivalentes Metall T enthält, aus der Gruppe, die durch Bor, Gallium, Aluminium und Eisen gebildet wird, eine Quelle für Fluoridionen F$^-$ und mindestens ein Strukturagens, das organische Kationen liefert, die Stickstoff enthalten und aus der Gruppe ist, die durch primäre und sekundäre aliphatische Monoamine, Diamine und Triamine gebildet wird und den Ammoniumkationen, die durch Protonierung der Mono-, Di-, Triamine entstehen, wobei das Agens eine Gesamtzahl von Kohlenstoffatomen und Stickstoffatomen von 3 bis 8 besitzt, wobei die Mischung eine Zusammensetzung aufweist, die bezogen auf das Molverhältnis zwischen den folgenden Wertebereichen liegt:

Si$^{IV}$/$\Sigma$T$^{III}$ 2-400 (T$^{III}$ = B, Al, Ga, Fe)

F$^-$/Si$^{IV}$ 0,1-3

Organisches Strukturelement / Si$^{IV}$ 0,1-4

H$_2$O/Si$^{IV}$ 4-200

b) daß man die Mischung bei einer Erwärmungstemperatur von über 270°C hält, um Kristalle zu erhalten, und

c) daß man diese Kristalle bei einer Temperatur von über 400°C kalziniert.

5. Verfahren nach Anspruch 4,

bei dem die Mischung in wässriger Lösung einen pH und eine Zusammensetzung, bezogen auf das Molverhältnis aufweist, die innerhalb der folgenden Bereiche liegen:

PH 4,5-9

Si$^{IV}$/$\Sigma$T$^{III}$ 4-150 (T$^{III}$ = B, Al, Ga, Fe)

F$^-$/Si$^{IV}$ 0,25-2,5

Organisches Strukturagens /Si$^{IV}$ 0,25-2,5

H$_2$O/Si$^{IV}$ 6-80.

6. Verfahren nach einem der Ansprüche 4 und 5,

bei dem die Erwärmungstemperatur der Reaktionsmischung zwischen 100 und 260°C gehalten wird.

7. Verfahren nach einem der Ansprüche 4 bis 6,

bei dem man die besagten Kristalle bei einer Temperatur zwischen 500 und 600°C kalziniert.

8. Verfahren nach einem der Ansprüche 4 bis 7,

bei dem die besagten Kristalle in Gegenwart eines Gases, das molekularen Sauerstoff enthält, kalziniert werden.

9. Verfahren nach einem der Ansprüche 4 bis 8,

bei dem man mindestens einen Teil der originalen Kationen durch Ionenaustausch gegen ein Kation oder eine Mischung von Kationen ersetzt, die aus den Gruppen IIa, IIIa, IVa, Ib, IIb, IIIb, IVb, VIb, VIII des Periodensystems der Elemente ausgewählt werden.

10. Verwendung eines Ferrierits oder eines Katalysators, der einen Ferrierit nach einem der Ansprüche 1 bis 3 enthält oder der nach einem der Ansprüche 4 bis 9 präpariert wurde, in einem Oligomerisierungsprozeß von Olefinen.